# EUROPÄISCHE PATENTANMELDUNG

(11) **EP 1 080 704 A1**
(43) Veröffentlichungstag der Anmeldung: **07.03.2001**
(21) Anmeldenummer: 00810700.5
(22) Anmeldetag: 04.08.2000
(51) Int. Cl.: A61F 2/46, A61F 2/36

(54) **Mittel zum Verbinden von modularen Teilen einer implantierbaren Prothese**

(30) Priorität: 03.09.1999 EP 99810795
(71) Anmelder: Sulzer Orthopedics Ltd., 6340 Baar (CH)
(72) Erfinder: Moses, Keren, Urbana, IL 61801 (US); Klaette, Markus, 01069 Dresden (DE); Mohamed, Hisham, Minneapolis, MN 55414 (US); Lahm, Ryan P., Big Lake, MN 55309 (US); Armbruster, Ryan, Minneapolis, MN 55401 (US); Zuckermann, Debbie, St. Paul, MN 55118 (US); Lanz, Martin, St. Paul, MN 55108 (US); Erdman, Arthur G., New Brighton, MN 55112 (US); Durfee, William K., Edina, MN 55439 (US); Gross, Walter, 8475 Ossingen (CH)
(74) Vertreter: Sulzer Management AG

(57) **Zusammenfassung**

Das Verbindungsmittel (1) ermöglicht das Verbinden von modularen Teilen (30,31) einer in der Orthopädie verwendeten implantierbaren Prothese (3). Dieses Verbindungsmittel (1) umfasst einen Schaft (10), der sich in einer Längsrichtung erstreckt, und an diesem Schaft (10) ein Kopfteil (12) sowie ein Gewinde (11). Das Gewinde (11) ist zum Einschrauben in einen ersten Prothesemodul (30) und der Schaft (10) zum Herstellen einer Verbindung zu einem zweiten Prothesemodul (31) vorgesehen. Dabei steht der Schaft (10) in seiner Längsrichtung unter einer Zug- oder Druckkraft, deren Betrag in einem vorgegebenen Intervall liegt. Ein Zusatzteil (13) ist mit dem Kopfteil (12) verbunden oder mit diesem verbindbar. Mit dem Zusatzteil (13) ist ein Drehmoment auf den Schaft (10) ausübbar. Das Zusatzteil (13) ist aufgrund von mindestens einer Sollbruchstelle (14), die in der Verbindung zum Kopfteil (12) angeordnet ist, von dem Kopfteil (12) trennbar.

## Beschreibung

Die Erfindung betrifft ein Mittel zum Verbinden von modularen Teilen einer in der Orthopädie verwendeten implantierbaren Prothese gemäss Oberbegriff von Anspruch 1, eine Montagevorrichtung für das Verbindungsmittel sowie Verwendungen dieses Verbindungsmittels.

Aus der EP-A 0 649 642 (= P.6603) ist eine modulare Femurkopfprothese bekannt, bei der ein distaler Schaftteil auf einer proximalen Endpartie mittels einer Dehnschaftschraube befestigt wird. Die beiden Protheseteile sind durch Anziehen der Schraube mit einer Vorspannkraft gegeneinander verspannbar. Die Vorspannkraft ist durch das Anzugsdrehmoment der Schraube bestimmt. Bei Verwendung eines Drehmomentschlüssels zum Herstellen der Schraubverbindung muss das Anzugsdrehmoment auf einen vorbestimmten Höchstwert begrenzt werden.

Der Drehmomentschlüssel soll im Operationssaal (OP-Bereich) einsetzbar sein. Daher muss er sterilisierbar sein. Andererseits muss der Höchstwert für das Anzugsdrehmoment einstellbar sein; d. h. der Drehmomentschlüssel muss kalibrierbar sein. Dies bedingt bei bekannten Drehmomentschlüsseln, dass deren Teile, die für das Kalibrieren vorgesehen sind, gefettete Stellen aufweisen müssen. Die Erfordernisse einer Sterilisierbarkeit einerseits und einer Kalibrierbarkeit andererseits sind schwer in Einklang zu bringen. Es ist daher Aufgabe der Erfindung, eine praktikable Lösung anzugeben, wie sich modulare Teile einer in der Orthopädie verwendeten Prothese im OP-Bereich mittels einer Schraubverbindung miteinander verbinden lassen. Die erfindungsgemässe Lösung wird mit dem im Anspruch 1 definierten Verbindungsmittel ermöglicht, das erlaubt, auf die Verwendung eines einstellbaren Drehmomentschlüssels zu verzichten.

Das Verbindungsmittel ermöglicht das Verbinden von modularen Teilen einer in der Orthopädie verwendeten implantierbaren Prothese. Dieses Verbindungsmittel umfasst einen Schaft, der sich in einer Längsrichtung erstreckt, und an diesem Schaft ein Kopfteil sowie ein Gewinde. Das Gewinde ist zum Einschrauben in einen ersten Prothesemodul und der Schaft zum Herstellen einer Verbindung zu einem zweiten Prothesemodul vorgesehen. Dabei steht der Schaft in seiner Längsrichtung unter einer Zug- oder Druckkraft, deren Betrag in einem vorgegebenen Intervall liegt. Ein Zusatzteil ist mit dem Kopfteil verbunden oder mit diesem verbindbar. Mit dem Zusatzteil ist ein Drehmoment auf den Schaft ausübbar. Das Zusatzteil ist aufgrund von mindestens einer Sollbruchstelle, die in der Verbindung zum Kopfteil angeordnet ist, von dem Kopfteil trennbar.

Die abhängigen Ansprüche 2 bis 6 beziehen sich auf vorteilhafte Ausführungsformen des erfindungsgemässen Verbindungsmittels. Gegenstand des Anspruchs 7 ist eine Montagevorrichtung. Die Ansprüche 8 und 9 betreffen besondere Verwendungen des erfindungsgemässen Verbindungsmittels.

Nachfolgend wird die Erfindung anhand der Zeichnungen erläutert. Es zeigen:
- Fig. 1: eine Montagevorrichtung zum Verbinden zweier Teile einer modularen Femurkopfprothese,
- Fig. 2: dieselbe Montagevorrichtung in Richtung II, Fig. 1, gesehen, mit einer Seitenansicht auf ein erfindungsgemässes Verbindungsmittel, das über ein Zusatzteil oder Anziehelement an die Montagevorrichtung angekoppelt ist,
- Fig. 3: einen Längsschnitt durch das Zusatzteil der Fig. 2,
- Fig. 4: eine zweite Ausführungsform des erfindungsgemässen Verbindungsmittels,
- Fig. 5: dasselbe Verbindungsmittel mit einem durch Sollbruch abgetrennten oberen Teil,
- Fig. 6: eine modulare Femurkopfprothese nach dem Zusammenfügen seiner zwei Module,
- Fig. 7: eine Demontage-Hülse,
- Fig. 8: einen Querschnitt durch die Hülse der Fig. 7, und
- Fig. 9: ein Zusatzteil einer weiteren Ausführungsform des erfindungsgemässen Verbindungsmittels

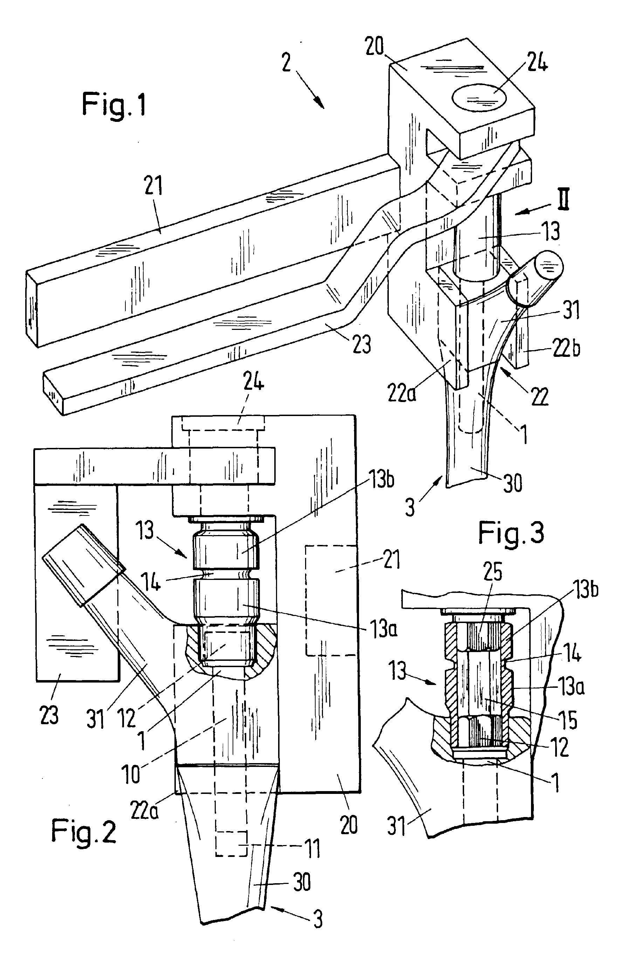

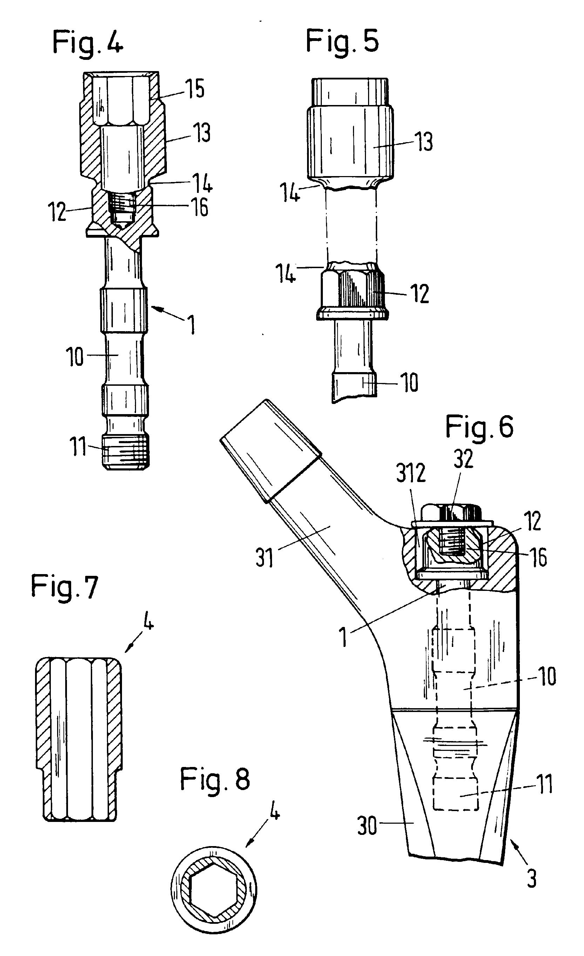

Mit der in den Figuren 1 und 2 dargestellten Montagevorrichtung 2 und einem erfindungsgemässen Verbindungsmittel 1 lassen sich bei einer modularen Femurkopfprothese 3 ein erster und ein zweiter Modul, nämlich ein distales Schaftteil 30 bzw. eine proximale Endpartie 31, zusammenspannen. Das Verbindungsmittel 1 umfasst einen Schaft 10 und einen Kopfteil 12 sowie ein Gewinde 11 (vgl. Fig. 3, 4). Das Gewinde 11 ist in den Prothesenmodul 30 eingeschraubt. Der Kopfteil 12 bildet eine Verankerung im zweiten Prothesenmodul 31. Die Montagevorrichtung 2 umfasst einen Grundkörper 20 mit einem ersten Hebel 21 und zwei Backen 22a und 22b, in deren Zwischenraum 22 die Endpartie 31 der Prothese 3 formschlüssig einlegbar ist. Ein zweiter Hebel 23 ist mit einem Zapfen 24 im Grundkörper 20 schwenkbar gelagert. Durch Verschwenken des Hebels 23 lässt sich ein Drehmoment auf das Verbindungsmittel 1 über ein Anziehelement oder Zusatzteil 13 (vereinfacht dargestellt) ausüben.

Das Zusatzteil 13 ist in Fig. 2 als Seitenansicht und in Fig. 3 als Querschnitt detaillierter dargestellt: Es ist rohrförmig, und sein Lumen 15 hat die Form eines senkrechten, sechsseitigen Prismas; es weist zwischen zwei Teilen 13a und 13b eine Ringnut 14 auf, die als Sollbruchstelle ausgebildet ist. In das Lumen 15 passen formschlüssig der als Sechskant geformte Kopfteil 12 des Verbindungsmittels 1 und ein ebenfalls als Sechskant geformter Teil 25, der mit dem Lagerzapfen 24 starr verbunden ist. Durch ein Bewegen der beiden Hebel 21 und 23 gegeneinander lässt sich auf das Zusatzteil 13 ein Drehmoment ausüben, das bei Überschreiten eines vorgebbaren Wertes zu einem Bruch längs der Nut 14 führt, so dass die beiden Teile 13a und 13b getrennt werden.

Die Sollbruchwerte der Nut 14 lassen sich bis auf ± 5% vorgeben, wobei lediglich normale Herstelltoleranzen (Drehen mit feiner Oberfläche, Rauhigkeit rund N6) erforderlich sind. Als Material für das Zusatzteil 13 eignen sich dabei Werkstoffe, die bei der Herstellung von chirurgischen Instrumenten üblicherweise verwendet werden. Das Verbindungsmittel 1, das als Dehnschaftschraube ausgebildet ist, muss aus einem zähelastischen Werkstoff hergestellt sein. Für das separate Zusatzteil 13 wird mit Vorteil ein etwas spröderer Werkstoff verwendet, für den der Bruch weitgehend verformungslos ist. Bei gleichem Werkstoff für das Verbindungsmittel 1 und die Prothese 3 kann der Bruch mittels Spannungsspitzen bewirkenden Kerben ausgelöst werden.

Beim Ausführungsbeispiel der Fig. 4 bilden das Zusatzteil 13 und der Schaft 10 ein monolithisches, aus einem einzigen Werkstoff hergestelltes Gebilde. Die Sollbruchstelle 14, die sich am Übergang vom Zusatzteil 13 zum Kopfteil 12 befindet, ist in das erfindungsgemässe Verbindungsmittel 1 integriert. Der Kopfteil 12, der Schaft 10 und das Gewinde 11 bilden im dargestellten Beispiel eine Dehnschaftschraube (siehe die oben genannte EP-A 0 649 642). Der Kopfteil 12 enthält ein Innengewinde 16.

Fig. 5 zeigt das vom Kopfteil 12 bei der Sollbruchstelle 14 abgetrennte Zusatzteil 13. Fig. 6 zeigt die Verbindung der zwei Module 30 und 31. Es ist auch gezeigt, dass ein abdeckendes Element 32 auf den Kopfteil 12 unter Verwendung des Innengewindes 16 aufgeschraubt ist.

Das im Modul 31 verankerte Kopfteil 12 ist von einem leeren, ringspaltförmigen Raum 312 umgeben. Dieser Raum 312 ermöglicht es, mit einer Demontage-Hülse 4, die in Fig. 7 als Längsschnitt und in Fig. 8 als Querschnitt abgebildet ist, die Verbindung zwischen den beiden Modulen - wieder unter Verwendung der Montagevorrichtung - zu lösen.

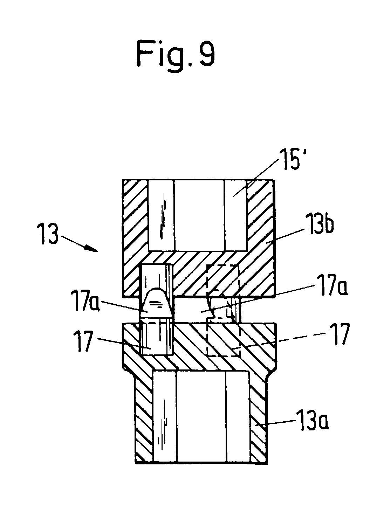

Das Ausführungsbeispiel der Fig. 9 zeigt ein Zusatzteil 13, dessen zwei Hälften 13a und 13b aus einem Kunststoff gefertigt sind und die über Stifte 17 miteinander verbunden sind. Diese Stifte 17 bestehen mit Vorteil aus einem spröden Material. Keilförmige Kerben 17a bilden Sollbruchstellen. Dem Lumen 15 bei der ersten Ausführungsform des erfindungsgemässen Verbindungsmittels entspricht hier ein Sackloch 15' mit einem prismatischen Innenraum. Es sind selbstverständlich auch andere Schwächungen als keilförmige Kerben denkbar, mit denen eine Sollbruchstelle festlegbar sind.

Beim Verbindungsmittel 1 für die modulare Femurkopfprothese 3 übt der Schaft 10 in seiner Längsrichtung eine Zugkraft aus. Das erfindungsgemässe Verbindungsmittel 1 kann bei anderen Ausführungsformen auch eine Druckkraft ausüben, beispielsweise bei Fixierelementen für Bänderprothesen.

## Patentansprüche

1. Mittel (1) zum Verbinden von modularen Teilen (30, 31) einer in der Orthopädie verwendeten implantierbaren Prothese (3), welches Verbindungsmittel (1) einen Schaft (10), der sich in einer Längsrichtung erstreckt, und an diesem Schaft ein Kopfteil (12) sowie ein Gewinde (11) umfasst, wobei das Gewinde zum Einschrauben in einen ersten Prothesemodul (30) und der Schaft zum Herstellen einer Verbindung zu einem zweiten Prothesemodul (31) vorgesehen sind, derart, dass der Schaft in seiner Längsrichtung unter einer Zug- oder Druckkraft steht, deren Betrag in einem vorgegebenen Intervall liegt,
dadurch gekennzeichnet, dass mit dem Kopfteil ein Zusatzteil (13) verbunden ist oder mit diesem verbindbar ist, mit dem ein Drehmoment auf den Schaft ausübbar ist, wobei das Zusatzteil aufgrund von mindestens einer Sollbruchstelle (14) in der Verbindung zum Kopfteil von diesem trennbar ist.

2. Mittel nach Anspruch 1, dadurch gekennzeichnet, dass im Falle, dass mit dem Schaft (10) eine Zugkraft auszuüben ist, der Kopfteil (12) eine Verankerung im zweiten Prothesenmodul (31) bildet.

3. Mittel nach Anspruch 1 oder 2, dadurch gekennzeichnet, dass der Kopfteil (12), der Schaft (10), das Zusatzteil (13) und die Verbindung (14) zum Kopfteil aus dem gleichen Material, insbesondere aus einer metallischen Legierung gefertigt sind.

4. Mittel nach Anspruch 1 oder 2, dadurch gekennzeichnet, dass der Kopfteil (12) und der Schaft (10) aus einem ersten Material, während das Zusatzteil (13, 13a, 13b) und die Verbindung (13a) zum Kopfteil aus einem zweiten Material gefertigt sind, wobei insbesondere das zweite Material spröder als das erste Material ist.

5. Mittel nach Anspruch 1 oder 2, dadurch gekennzeichnet, dass die Verbindung (13a, 17) zwischen Kopfteil (12) und Zusatzteil (13) aus mindestens einem separat hergestellten Teil gebildet ist, durch dessen Abmessungen und Materialeigenschaften die Widerstandskraft der Sollbruchstelle vorgegeben ist, wobei insbesondere mittels einer Nut (14) oder Kerbe (17a) die Widerstandskraft mitbeeinflusst ist.

6. Mittel nach einem der Ansprüche 1 bis 5, dadurch gekennzeichnet, dass ein Demontage-Mittel (4) vorgesehen ist, mit denen nach einem Herstellen der Verbindung zwischen den Prothesemodulen (30, 31) und Abtrennen des Zusatzteils (13) ein Lösen der Verbindung durchführbar ist.

7. Montagevorrichtung (2) zum Verbinden eines ersten Moduls (30) mit einem zweiten Modul (31) bei einer modularen Prothese (3), mittels eines Verbindungsmittel (1) gemäss einem der Anspüche 1 bis 6, folgende Komponenten umfassend: einen Grundkörper (20) mit einem ersten Hebel (21) und zwei Backen (22a, 22b), zwischen welche ein Teil des zweiten Moduls formschlüssig einlegbar ist, und einen zweiten Hebel (23), der im Grundkörper schwenkbar gelagert ist und mit dem ein Drehmoment über ein Zusatzteil (13) auf das Verbindungsmittel ausübbar ist.

8. Verwendung eines Verbindungsmittels (1) gemäss einem der Anspüche 1 bis 6, zum Befestigen eines distalen Schaftteils (30) an einer proximalen Endpartie (31) bei einer modulare Femurkopfprothese (3).

9. Verwendung eines Verbindungsmittels gemäss einem der Anspüche 1 bis 6 als Fixierelemente bei Bänderprothesen.
